(19) Europäisches Patentamt
European Patent Office
Office européen des brevets

(11) **EP 3 055 033 B1**

(12) **EUROPEAN PATENT SPECIFICATION**

(45) Date of publication and mention
of the grant of the patent:
**11.04.2018 Bulletin 2018/15**

(51) Int Cl.:
***A61Q 15/00*** (2006.01)     ***A61K 8/26*** (2006.01)
***A61K 8/92*** (2006.01)     ***A61K 8/06*** (2006.01)

(21) Application number: **14790504.6**

(22) Date of filing: **08.10.2014**

(86) International application number:
**PCT/DK2014/050322**

(87) International publication number:
**WO 2015/051805 (16.04.2015 Gazette 2015/15)**

(54) **IMPROVED ANTIPERSPIRANT COMPOSITION**

VERBESSERTE SCHWEISSHEMMENDE ZUSAMMENSETZUNG

COMPOSITION ANTIPERSPIRANTE AMELIORÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.10.2013 DK 201370568**

(43) Date of publication of application:
**17.08.2016 Bulletin 2016/33**

(73) Proprietor: **Riemann Trading ApS
3400 Hillerød (DK)**

(72) Inventor: **BRITZE, Lene
2980 Kokkedal (DK)**

(74) Representative: **Plougmann Vingtoft a/s
Rued Langgaards Vej 8
2300 Copenhagen S (DK)**

(56) References cited:
**WO-A1-00/10521          WO-A1-89/02264
WO-A1-2007/073740     WO-A2-00/10512
US-A- 4 526 780          US-A- 5 162 378
US-A1- 2007 020 211**

**Description**

**Technical field of the invention**

The present invention relates to antiperspirant compositions, methods of preventing or reducing perspiration using such antiperspirant compositions and methods of manufacturing antiperspirant compositions. In particular the present invention relates to high efficacy antiperspirant compositions with further improved skin feel comprising aluminium chloride, a calcium or magnesium salt of an organic acid and a water miscible solvent.

**Background of the invention**

High efficacy antiperspirant compositions are well known in the art and generally comprise relatively high concentrations of specific multivalent metal salts which provide the antiperspirant effect. Aluminium and aluminium-zirconium antiperspirant salts are commonly employed, particularly chlorohydrate salts thereof, such as aluminium chlorohydrate (e.g. $Al(OH)_2Cl_4$) or a mixture of aluminium chlorohydrate and zirconium chlorohydrates.
Aluminium chloride ($AlCl_3$) is also well known antiperspirant salt, but the use of aluminium chloride is limited mainly due to the fact that aluminium chloride liberates aluminium chlorohydrate as well as hydrochloric acid upon contact with water. Therefore, compositions comprising aluminium chloride should preferably be anhydrous to avoid undesirable release of hydrochloric acid in the composition prior to use. Furthermore, the release of hydrochloric acid upon contact with water present in the skin of the user also present problems with the skin feel of aluminium chloride compositions, and buffer components should be added to reduce irritation to a minimum due to the hydrochloric acid.
Thus, WO 2007/073740 discloses antiperspirant compositions comprising aluminium chloride in an alcohol-in-oil type emulsion, where aluminium lactate is used as a buffer component. As will be shown herein the skin feel properties can be even further improved in comparison to such compositions.
In EP 1105087 antiperspirants compositions comprising aluminium chlorohydrate salts and calcium chloride are disclosed. It is claimed that the calcium chloride salts improve the thermal efficacy of the antiperspirant compositions, however, nothing is taught regarding the skin feel.
Also, US 3,998,788 discloses the use of trace amounts of calcium or magnesium salts to form complexes with aluminium or aluminium-zirconium chlorohydrates for use in antiperspirant compositions. The presence of small amounts of these salts is reported to enhance antiperspirant efficacy. The salts are however not salts of organic acids, nor are any effects with respect to skin feel reported.
Hence, an improved antiperspirant composition with high efficacy and further improved good skin feel properties would be advantageous, and in particular an aluminium chloride based antiperspirant composition with additives to even further improve skin feel while maintaining high efficacy would be advantageous.

**Summary of the invention**

Thus, an object of the present invention relates to improved high efficacy antiperspirant compositions based on aluminium chloride dissolved in a water miscible solvent, wherein said compositions have further improved skin feel properties while maintaining high antiperspirant efficacy.
In particular, it is an object of the present invention to provide an aluminium chloride antiperspirant that further solves the above mentioned problems of the prior art with irritation on the skin caused by the formation of hydrochloric acid upon contact of aluminium chloride with water.
The present inventors have surprisingly found that the addition of at least one calcium or magnesium salt of an organic acid to anhydrous antiperspirant compositions comprising aluminium chloride leads to further improved skin feel as compared to using other salts, such as for example aluminium salts of organic acids, while maintaining the high efficacy of the antiperspirant. This improved skin feel and reduction in irritation could surprisingly be provided even when increasing the amount of aluminium chloride in the composition as compared to the prior art.
Thus, one aspect of the invention relates to an anhydrous antiperspirant composition comprising:

    i) $AlCl_3$ or any hydrates thereof,
    ii) at least one calcium or magnesium salt of an organic acid, and
    iii) at least one water miscible solvent.

Another aspect of the present invention relates to a method of preventing or reducing perspiration comprising applying to the skin an anhydrous antiperspirant composition comprising:

    i) $AlCl_3$ or any hydrates thereof,

2

ii) at least one calcium or magnesium salt of an organic acid, and
iii) at least one water miscible solvent.

Yet another aspect of the present invention is to provide a method of manufacturing an anhydrous antiperspirant composition comprising:

I) providing at least one water miscible solvent,
II) optionally adding a thickening agent,
III) Adding at least one calcium or magnesium salt of an organic acid,
IV) optionally adding an oil phase,
V) Adding a solution of AlCl$_3$ or any hydrates thereof,

to form said anhydrous antiperspirant composition.

**Brief description of the figures**

Figure 1 shows a composition consisting of ethanol and aluminium chloride, i.e. an aluminum chloride solution in ethanol.

Figure 2 shows a composition consisting of hydroxypropylcellulose in ethanol.

Figure 3 shows a composition consisting of ethanol, hydroxypropylcellulose and calcium lactate.

Figure 4 shows a composition consisting of ethanol, hydroxypropylcellulose, calcium lactate and aluminium chloride.

The present invention will now be described in more detail in the following.

**Detailed description of the invention**

Definitions

Prior to discussing the present invention in further details, the following terms and conventions will first be defined:

In the present context an *"anhydrous"* composition means a composition which is essentially free of water. Particularly, for such a composition significant amounts of water has not been added, and the amount of un-bound water in the composition is very low. Some components in an antiperspirant composition may include some bound water, i.e. water of crystallisation in the form of hydrates. This water is however not immediately available for participating in hydrolysis reactions with water sensitive components. In preferred embodiments the anhydrous composition of the present invention comprises less than 5%(w/w) water, such as less than 4%(w/w) water, 3%(w/w), 2%(w/w), 1.5%(w/w), 1%(w/w), 0.5%(w/w), such as less than 0.2% (w/w) water as compared to total composition weight. Preferably the amount of added water in an anhydrous composition is less than 3%(w/w), such as less 2%(w/w), 1%(w/w), 0.5%(w/w), such as less than 0.1%(w/w).

In the present context *"antiperspirant"* composition is a composition comprising active ingredients or precursors thereof capable of topically reducing sweat production in mammals, particularly humans. All components in an antiperspirant composition or deodorant must be cosmetologically acceptable, i.e. they must be approved for topical application to the skin in the concentrations used.

In the present context *"aluminium chloride"* is used interchangeably with AlCl$_3$. The term also includes hydrates of AlCl$_3$, such as aluminium chloride hexahydrate. Aluminium chloride is a well-known antiperspirant salt, which forms aluminium chlorohydrate and hydrochloric acid upon contact with water present in the skin of the user. Aluminium is referred to as "aluminum" in some countries.

In the present context an *"organic acid"* is an acid derived from an organic molecule. Such acids may include carboxylic acids, anhydrides of carboxylic acids, sulfonic, and phosphoric acids. The organic acid may include other functional groups than the carboxylic acid, and may include amino acids. The acid must be able to form a salt with magnesium or calcium ions in the present context.

In the present context *"water miscible solvent"* is a solvent which may be mixed with water without any phase separation. Water miscible solvents particularly include hydrophilic solvents. Such solvents include for example lower alkyl alcohols and hydrophilic organic solvents.

In the present context *"percent weight/weight"* or *"%(w/w)"* are used interchangeably and indicates the weight percentage of the aforementioned component or ingredient as compared to total composition weight, i.e. the weight of the finished composition unless otherwise indicated. In certain cases it may refer to the weight percentage as compared to an intermediate composition.

In the present context *"alkyl alcohol"* is in the broadest sense an organic alcohol consisting of a branched or linear alkyl chain and one or more hydroxyl groups attached thereto.

Having made the above definitions, a first aspect of the present invention is an anhydrous antiperspirant composition comprising

> i) $AlCl_3$ or any hydrates thereof,
> ii) at least one calcium or magnesium salt of an organic acid, and
> iii) at least one water miscible solvent.

The organic acid provides a buffer effect with respect to the hydrochloric acid formed when e.g. applying aluminium chloride to the skin. Thus, preferably the organic acid is a relatively weak organic acid, and always weaker than hydrochloric acid. Thus, in a preferred embodiment the organic acid is an acid with a pKa value in the range of 2.0-15.0, such as 2.0-12.0, 2.0-10.0, 2.0-8.0, 2.0-6.0, such as 2.3-5.0.

A range of organic acids are suitable for use in the present topical compositions and are weaker than hydrochloric acid. This include organic carboxylic acids, anhydrides thereof, amino acids and weak organic phosphoric acids. Thus in a preferred embodiment said organic acid is selected from the group consisting of citric, formic, acetic, gluconic, ascorbic, lactic, propionic, acetylsalicylic, benzoic, salicylic, nicotinic, tartaric, phtalic acid, fatty acids such as oleic, linoleic, undecenoic, octanoic, palmitic, ricinoleic, and, stearic, acid, acetylcretosinic, succinic, carbamoylphenoxyacetic, diacetylsalicylic, anthranilic, mefenamic, gentisic, tolfenamic, acetotartaric, agaric, subacetic, ellagic, fumaric, malic, morrhuic, oxalic, para-amino-benzoic, gallic, cinnamic, isoascorbic, sorbic, aminocaproic, aminomethylbenzoic, and tranxenamic acid, and also naturally occuring amino acids such as glycine, alanine, valine, leucine, isoleucine, serine and threonine.

Particularly preferred organic acids are selected from the group consisting of acetylsalicylic, salicylic, benzoic, propionic, octanoic, undecanoic, sorbic acid, ascorbic acid, lactic acid, malic acid, stearic acid, citric acid, phthalic acid, tartaric acid, or a naturally occurring amino acid. The most preferred organic acid is lactic acid.

The inventor has surprisingly found that a specific choice of counter-ion to the organic acid provides for further improved skin feel and further reduced skin irritation of the compositions while the efficacy may be maintained as compared to prior art composition, where aluminium salts of organic acids are used. This effect is seen even though the amount of irritant, i.e. aluminium chloride, is increased in the novel compositions of the present invention as compared to prior art compositions. Thus, calcium or magnesium salts of the organic acids surprisingly provides for the effects described above. Particularly preferred are calcium salts of the organic acid. The most preferred salt of an organic acid is calcium lactate. It should be noted that compositions comprising both calcium and magnesium salts of the organic acids are part of this invention.

The term "skin feel" and "sensory skin feeling" are used interchangeably and are intended to mean how various subjective skin feeling characteristics are perceived by a human user e.g. such as how new compositions of the invention and the existing type composition feels neutral and/or comfortable to the skin; feels greasy and/or sticky on the skin; induces skin irritation.

The term "skin irritation" is meant to mean e.g. itching of the skin, a stinging and/or burning sensation of the skin and redness and/or erythema of the skin.

In order to prevent premature reaction of the aluminium chloride with water, it is a part of the present invention that the composition is anhydrous. This is in particular obtained by using a carrier system, which is anhydrous or has no available water so that aluminium chloride does not undergo hydrolysis to aluminium chlorohydrate and hydrochloric acid.

The terms "no available water" and "substantially free from water" are used interchangeably and are intended to mean that the water present in the formulation is not able to solvate the aluminium salts, either because the water is present in too small an amount, or because the water is bound too strongly by another component in the composition. Thus, $AlCl_3$ will for example not undergo hydrolysis in 95% ethanol or in liquid sorbitol.

The compositions of the present invention are anhydrous, however a water miscible solvent is used which dissolves the components of the composition, in particular the aluminium chloride salts and the salt of the organic acid. The solution provided may be an emulsion due to the presence of the optional oil phase, however no significant amount of solid particles are present in the compositions of the present invention. This is an advantage, as sedimentation is avoided. From example 6 it is clear that the composition of the present invention is a solution or an emulsion but not a suspension. Thus, in a preferred embodiment the water miscible solvent is selected from the group consisting of a $C_2$-$C_5$ alkyl alcohol, glycerol, and $C_2$-$C_5$ glycol. These solvents provides for stable solutions or emulsion without significant sedimentation. In a particularly preferred embodiment the water miscible solvent is a $C_2$-$C_5$ alkyl alcohol. Even more preferred the $C_2$-$C_5$ alkyl alcohol is selected from the group consisting of ethanol, 1-propanol, 2-propanol, ethyleneglycol, propylene glycol, 1-butanol, and 2-butanol. The most preferred solvent is ethanol. Ethanol in particular may be denatured. To minimize the addition of unbound water absolute ethanol may be used.

The antiperspirant salt used is aluminium chloride, which may be provided as $AlCl_3$ or any hydrates thereof. Particularly useful versions of this salt may be selected from the group consisting of aluminium chloride or aluminium chloride hexahydrate.

The compositions of the present invention may be in the form of an emulsion if an oil phase is added. Thus, in a preferred embodiment of the present invention a composition is provided, wherein said composition comprises an oil phase. The oil phase may preferably comprise a hydrogenated vegetable oil. The oil phase may even more preferably consist of a hydrogenated vegetable oil. In a preferred embodiment said hydrogenated vegetable oil is hydrogenated castor oil.

The viscosity or thickness of the compositions herein may be adjusted using thickening agents. Therefore, in a preferred embodiment said composition comprises a thickening agent. Said thickening agent may be selected from the group consisting of hydroxyethylcellulose, hydroxypropylcellulose, silica, hectorite, such as stearalkonium hectorite, and any mixtures thereof. Although the thickening agents may be used in various compositions, particularly preferred for roll-on compositions are hydroxyethylcellulose and hydroxypropylcellulose thickening agents, most preferably hydroxypropylcellulose. On the other hand for stick-gel formulations hectorite type thickening agents are particularly preferred.

Since the amount of the irritant hydrochloric acid produced when the present composition of aluminium chloride is contacted with excess water is dependent on the amount of aluminium chloride, it is preferred to have a certain ratio between the aluminium and the organic acid which provides a buffer effect to reduce irritation. Thus, in a preferred embodiment a composition according to the present invention is provided wherein the molar ratio of the organic acid to aluminium (acid:aluminium) is in the range of 1:1.13-1:2.00, such as 1:1.13-1:1.90, 1:1.13-1:1.80, 1:1.13-1:1.70, 1:1.13-1:1.60, 1:1.13-1:1.50, 1:1.13-1:1.40, 1:1.13-1:1.35, 1:1.13-1:1.30, 1:1.13-1:1.20, 1:1.13-1:1.18, such as preferably in the range of 1:1.13-1:1.15.

The antiperspirant salt must be present in an amount to provide the desired antiperspirant efficacy. The desired efficacy may vary, however in the context of the present invention a composition is preferred wherein the $AlCl_3$ or hydrate thereof is present in an amount in the range of 5-25%(w/w), such as in the range of 6-22%(w/w), 7-20%(w/w), 8-19%(w/w), 9-18%(w/w), 10-17%(w/w), 11-17%(w/w), 12-17%(w/w), such as in the range of 13-17%(w/w) as compared to total composition weight.

The salt of the organic acid must be present in an amount to provide the desired skin feel properties, which again is dependent on the amount of aluminium chloride in the compositions - and any further acid irritants. Thus, in preferred embodiments said calcium or magnesium salt of an organic acid is present in an amount in the range of 1-25%(w/w), such as in the range of 2-20%(w/w), 4-18%(w/w), 5-16%(w/w), 6-15%(w/w), 7-14%(w/w), 8-13%(w/w), 9-12%(w/w), such as in the range of 10-12%(w/w) as compared to total composition weight.

The water miscible solvent of the present invention may be present in an amount in the range of 5-90%, such as in the range of 10-90%(w/w), 15-90%(w/w), 20-90%(w/w), 30-90%(w/w), 40-90%(w/w), 45-85%(w/w), 50-85%(w/w), 55-85%(w/w), 60-85%(w/w), 65-85%(w/w), 70-80%(w/w), such as in the range of 72-76%(w/w) as compared to total composition weight. In the context of the present invention the solvent should preferably be present in an amount to at least dissolve the added components, while further solvent may be added to adjust the concentrations of the various components to a suitable level.

The solvent may be combined with a thickening agent and other additives to provide a suitable carrier material for the antiperspirant composition. Thickening agents and amounts thereof may vary according to the desired formulation type, and according to which solvent is used. In an preferred embodiment said thickening agent is present in an amount in the range of 0.01-20%(w/w), such as in the range 0.05-18%(w/w), 0.08-15%(w/w), 0.10-12%(w/w), 0.12-10%(w/w), 0.15-8%(w/w), 0.17-5%(w/w), 0.19-2%(w/w), such as in the range of 0.20-0.1%(w/w) as compared to total composition weight.

As mentioned, an oil-phase may be added to provide an emulsion. In preferred embodiments said oil phase is present in an amount in the range of 0.001-1.0%(w/w), such as in the range 0.005-0.80%(w/w), 0.01-0.70%(w/w), 0.02-0.70%(w/w), 0.03-0.70%(w/w), such as in the range of 0.04-0.70%(w/w) as compared to total composition weight.

Although a combination of solvents may be used, according to the present invention it is preferred that the water miscible solvent is the main solvent and thus a composition is preferred where the water miscible solvent or a combination of water miscible solvents form from 80-100%(w/w) of the total amount of solvents, such as from 85-100%(w/w), 90-100%(w/w), 95-100%(w/w), 98-100%(w/w), 99-100%(w/w), 99.9-100%(w/w), such as 100%(w/w) of the total amount of solvents. Preferably the $AlCl_3$ or any hydrates thereof and the calcium or magnesium salt of an organic acid are dissolved in the solvent. Even more preferably the composition of the invention is a solution. The solution may be in the form of an emulsion if an oil phase is present, but preferably the emulsion is particle free.

In another embodiment the composition of the invention comprises a filler. The filler may preferably be selected from the group consisting of starch, talc and derivatives or mixtures thereof.

In yet another embodiment the composition of the present invention comprises a first emollient. Said first emollient may comprise a siloxane derivative. Said siloxane derivative may be cyclopentasiloxane. Preferably said first emollient is present in an amount in the range of 1-9%(w/w), such as 2-8%(w/w), such as 3-7%(w/w), such as 4-6%(w/w) as compared to total composition weight.

In yet another embodiment the composition of the present invention comprises a second emollient, and preferably said second emollient is a cetyl ester, such as cetyl palmitate. Preferably, said second emollient is present in an amount of less than 5%(w/w), such as less than 4%(w/w), such as less than 3%(w/w), such as less than 2%(w/w), such as less than 1%(w/w) as compared to total composition weight.

In a preferred embodiment the composition of the present invention comprises an emulsifier. Said emulsifier may be a glyceryl ester, such as preferably glyceryl stearate. Preferably, said emulsifier is present in an amount of less than 5%(w/w), such as less than 4%(w/w), such as less than 3%(w/w), such as less than 2%(w/w), such as less than 1%(w/w) as compared to total composition weight.

Preferably the composition of the present invention may be a composition having an efficacy which corresponds to an effectiveness where at least 50% of a group of efficacy test population experiences a more than 20% reduction in sweat production. Even more preferably the composition of the present invention may be a composition having an efficacy which corresponds to an extra effectiveness where at least 50% of a group of efficacy test population experiences a more than 30% reduction in sweat production. These criteria correspond to the "effectiveness" and "extra effectiveness" criteria of the FDA guidelines for antiperspirants, in accordance with § 350.60 (21 CFR 350.60) of the final monograph (final rule) for OTC antiperspirant drug products, published in the federal register on June 9, 2003.

Another aspect of the present invention is a method of preventing or reducing perspiration comprising applying to the skin an anhydrous antiperspirant composition comprising:

i) $AlCl_3$ or any hydrates thereof,
ii) at least one calcium or magnesium salt of an organic acid, and
iii) at least one water miscible solvent.

Yet another aspect of the present invention is a method of manufacturing an anhydrous antiperspirant composition comprising:

I) providing at least one water miscible solvent
II) optionally adding a thickening agent
III) Adding at least one calcium or magnesium salt of an organic acid
IV) optionally adding an oil phase
V) Adding a solution of $AlCl_3$ or any hydrates thereof

to form said anhydrous antiperspirant composition.

In a preferred embodiment the components under steps II) to V) are added under stirring. In another preferred embodiment said solution of $AlCl_3$ or any hydrates thereof comprises a water miscible solvent and $AlCl_3$.

An embodiment of the invention relates to an anhydrous antiperspirant composition comprising

i) $AlCl_3$ or any hydrates thereof in an amount of 5-25 %(w/w), such as 10-20 %(w/w), such as 12-18 %(w/w), such as 14-16 %(w/w),
ii) at least one calcium or magnesium salt of an organic acid in an amount of 1-25 %(w/w), such as of 5-15 %(w/w), such as 7-13 %(w/w), such as 8-11.5 %(w/w), and
iii) at least one water miscible solvent in an amount of 5-90 %(w/w), such as 70-80 %(w/w), such as 72-78 %(w/w).

An embodiment of the invention relates to an anhydrous antiperspirant consisting essentially of

i) $AlCl_3$ in an amount of 15 %(w/w),
ii) calcium lactate in an amount of 11 %(w/w), and
iii) ethanol in an amount of 73.7 %(w/w).

An embodiment of the invention relates to an anhydrous antiperspirant essentially consisting of

i) AlCl$_3$ in an amount of 15 %(w/w),
ii) calcium lactate in an amount of 11 %(w/w), and
iii) ethanol in an amount of 73.7 %(w/w).

An embodiment of the invention relates to an anhydrous antiperspirant consisting essentially of

i) AlCl$_3$ in an amount of about 15 %(w/w),
ii) calcium lactate in an amount of about 11 %(w/w), and
iii) ethanol in an amount of about 73.7 %(w/w).

An embodiment of the invention relates to an anhydrous antiperspirant essentially consisting of

i) AlCl$_3$ in an amount of about 15 %(w/w),
ii) calcium lactate in an amount of about 11 %(w/w), and
iii) ethanol in an amount of about 73.7 %(w/w).

A preferred embodiment of the invention relates to an anhydrous antiperspirant consisting of

i) AlCl$_3$ in an amount of about 15 %(w/w),
ii) calcium lactate in an amount of about 11 %(w/w),
iii) ethanol in an amount of about 73.7 %(w/w),
iii) hydroxypropylcellulose in an amount of about 0.25 %(w/w), and
iv) Hydrogenated Castor Oil in an amount of about 0.05 %(w/w).

An embodiment of the invention relates to an anhydrous antiperspirant consisting essentially of

i) AlCl$_3$ in an amount of about 15 %(w/w),
ii) magnesium lactate in an amount of about 8.5 %(w/w), and
iii) ethanol in an amount of about 76.21 %(w/w).

An embodiment of the invention relates to an anhydrous antiperspirant essentially consisting of

i) AlCl$_3$ in an amount of 15 %(w/w),
ii) magnesium lactate in an amount of about 8.5 %(w/w), and
iii) ethanol in an amount of about 76.21 %(w/w).

A preferred embodiment of the invention relates to an anhydrous antiperspirant consisting of

i) AlCl$_3$ in an amount of about 15 %(w/w),
ii) magnesium lactate in an amount of about 8.5 %(w/w),
iii) ethanol in an amount of about 76.21 %(w/w),
iii) hydroxypropylcellulose in an amount of about 0.23 %(w/w), and
iv) Hydrogenated Castor Oil in an amount of about 0.06 %(w/w).

The present invention also relates to the use of anhydrous antiperspirant according to the invention for improving sensory skin feel or skin feeling when the antiperspirant is applied to the skin of a in a human.
The present invention also relates to the use of anhydrous antiperspirant according to the invention for improving the natural and/or comfortable skin feel or skin feeling when the antiperspirant is applied to the skin of a in a human.
The present invention also relates to the use of anhydrous antiperspirant according to the invention for reducing greasy and/or sticky sensory feeling when the antiperspirant is applied to the skin of a human.
The present invention also relates to the use of anhydrous antiperspirant according to the invention for reducing skin irritation when the antiperspirant is applied to the skin of a human.
The present invention also relates to the use of anhydrous antiperspirant according to the invention for reducing itching when the antiperspirant is applied to the skin of a human.
The present invention also relates to the use of anhydrous antiperspirant according to the invention for reducing a stinging and/or burning sensation of the skin when the antiperspirant is applied to the skin of a human.
The present invention also relates to the use of anhydrous antiperspirant according to the invention for reducing redness and/or erythema of the skin when the antiperspirant is applied to the skin of a human.

The present invention also relates to a method of preventing or reducing a greasy and/or sticky sensory feeling comprising applying to the skin of a human an anhydrous antiperspirant according to the invention.

The present invention also relates to a method of preventing or reducing skin irritation comprising applying to the skin of a human an anhydrous antiperspirant according to the invention.

The present invention also relates to a method of preventing or reducing itching of the skin comprising applying to the skin of a human an anhydrous antiperspirant according to the invention.

The present invention also relates to a method of preventing or reducing a stinging and/or burning sensation of the skin comprising applying to the skin of a human an anhydrous antiperspirant according to the invention.

The present invention also relates to a method of preventing or reducing redness and/or erythema of the skin comprising applying to the skin of a human an anhydrous antiperspirant according to the invention.

The present invention also relates to an anhydrous antiperspirant for use in preventing or reducing perspiration comprising applying to the skin of a human an anhydrous antiperspirant according to the invention.

The present invention also relates to an anhydrous antiperspirant according to the invention for use in improving sensory skin feel or skin feeling when the antiperspirant is applied to the skin of a in a human.

The present invention also relates to an anhydrous antiperspirant according to the invention for use in improving the natural and/or comfortable skin feel or skin feeling when the antiperspirant is applied to the skin of a in a human.

The present invention also relates to an anhydrous antiperspirant according to the invention for use in reducing a greasy and/or sticky sensory feeling when the antiperspirant is applied to the skin of a human.

The present invention also relates to an anhydrous antiperspirant according to the invention for use in reducing skin irritation when the antiperspirant is applied to the skin of a human.

The present invention also relates to an anhydrous antiperspirant according to the invention for use in reducing itching when the antiperspirant is applied to the skin of a human.

The present invention also relates to an anhydrous antiperspirant according to the invention for use in reducing a stinging and/or burning sensation of the skin when the antiperspirant is applied to the skin of a human.

The present invention also relates to an anhydrous antiperspirant according to the invention for use in reducing redness and/or erythema of the skin when the antiperspirant is applied to the skin of a human.

It should be noted that embodiments and features described in the context of one of the aspects of the present invention also apply to the other aspects of the invention. Particularly, the preferred embodiments applying to the composition of the present invention also apply to the method of reducing perspiration and the method of manufacturing an antiperspirant.

All patent and non-patent references cited in the present application, are hereby incorporated by reference in their entirety.

The invention will now be described in further details in the following non-limiting examples.

**Examples**

Throughout examples 1 and 2 the compositions were mixed using a Silverson high speed mixer with emulsion screen. The ethanol used was absolute ethanol with denaturant. The aluminium chloride used was aluminium chloride hexahydrate. Bentone gel IPM V represents a 87:10:3 mixture of isopropyl myristate, stearalkonium hectorite and propylene carbonate respectively.

Example 1 - Antiperspirant roll-on formulations

Antiperspirant compositions according to the present invention were manufactured in the following way.

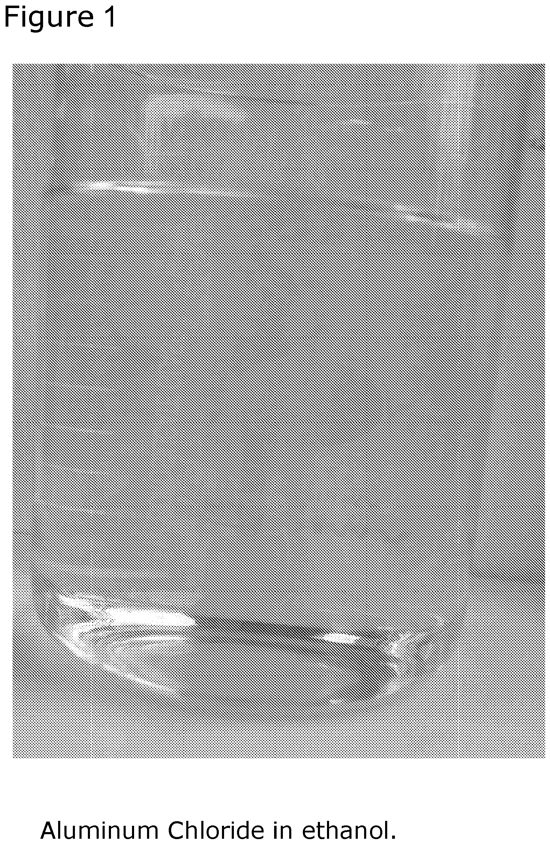

An aluminium chloride solution was prepared in a separate container to make a solution of aluminium chloride in ethanol. The solution was stirred up to 24h, until as much as possible of the powder was solubilized. The solution was filtered to remove any trace excess of aluminium chloride powder. Amounts used are given in table 1. The resulting aluminium chloride solution is a clear liquid, see Figure 1.

TABLE 1: ALUMINIUM CHLORIDE SOLUTION

| Raw material | Weight (g) | RPM | Time (min) | %(w/w) |
|---|---|---|---|---|
| Ethanol denatured | 525.00 | | | 75 |
| Aluminium Chloride | 175.00 | 2500 | 24h | 25 |
| Sum | 700.00 | | | 100 |
| RPM = rounds per minute. Time = the duration of stirring. | | | | |

EP 3 055 033 B1

To prepare the antiperspirant composition a 2 liter beaker was charged with ethanol and Hydroxypropylcellulose was gently sprinkled into the ethanol, while assuring moderate mixing. Calcium lactate was added and stirring was adjusted to insure good mixing. Hydrogenated castor oil was added, followed by addition of the Aluminium Chloride solution. Stirring was continued until a homogeneous mixture was obtained.

TABLE 2: ROLL-ON TYPE ANTIPERSPIRANT COMPOSITION COMPRISING CALCIUM LACTATE

| Raw material | Weight (g) | RPM | Time (min) | %(w/w) |
|---|---|---|---|---|
|  |  |  |  |  |
| Ethanol denatured | 287 |  |  | 28.7 |
| Hydroxypropylcellulose | 2.5 | 2500-4000 | 25 | 0.25 |
| Calcium lactate | 110 | 4000 | 4 | 11 |
| Hydrogenated Castor Oil | 0.5000 | 4000 | 3 | 0.05 |
| Aluminium Chloride solution | 600.00 | 4500 | 8 | 60 |
| sum | 1000.00 |  |  |  |
| RPM = rounds per minute. Time = the duration of stirring | | | | |

|  | Weight (g) | %(w/w) |
|---|---|---|
| Sum total ethanol | 737 | 73.7 |
| Sum total Aluminium Chloride | 150 | 15 |

The total sum of Aluminium Chloride in the composition depicted in Table 2 is 150 g corresponding to 15 %(w/w) as only 600 g of the finished Aluminium Chloride solution is used in the product as shown in Table 2. Accordingly, the total sum of ethanol in the composition depicted in table 2 is 737 g corresponding to 73.7 %(w/w), i.e. some of the ethanol is derived from the Aluminium Chloride solution shown in table 1.

A composition comprising magnesium lactate rather than calcium lactate may be manufactured in an analogous way as the above, according to the amounts given in tables 3 and 4 below.

TABLE 3: ALUMINIUM CHLORIDE SOLUTION

| Raw material | Weight (g) | %(w/w) |
|---|---|---|
| Ethanol denatured | 450.00 | 75 |
| Aluminium Chloride | 150.00 | 25 |
| sum | 600.00 | 100 |

TABLE 4: ROLL-ON TYPE ANTIPERSPIRANT COMPOSITION COMPRISING MAGNESIUM LACTATE

| Raw material | Weight (g) | %(w/w) |
|---|---|---|
| Ethanol denatured | 312.10 | 31.21 |
| Hydroxypropylcellulose | 2.30 | 0.23 |
| Magnesium lactate | 85.00 | 8.50 |
| Hydrogenated Castor Oil | 0.60 | 0.06 |
| Aluminium Chloride solution | 600.00 | 60.00 |
| Sum | 1000.00 | 100 |
|  |  |  |

(continued)

|  | Weight (g) | %(w/w) |
|---|---|---|
| Sum total ethanol | 762.1 | 76.21 |
| Sum total Aluminium Chloride | 150 | 15 |

The total sum of Aluminium Chloride in the composition depicted in Table 4 is 150 g corresponding to 15 %(w/w) as only 600 g of the finished Aluminium Chloride solution is used in the product as shown in Table 4. Accordingly, the total sum of ethanol in the composition depicted in table 4 is 762.1 g corresponding to 76.21 %(w/w), i.e. some of the ethanol is derived from the Aluminium Chloride solution shown in table 3.

Example 2 - Antiperspirant gel-stick formulations

Gel stick formulations were manufacture in a similar way as example 1, table 1. The aluminium chloride solution was made as explained in example 1. To achieve the gel-stick type formulations alternative or additional agents to the hydroxypropylcellulose thickening agents were used as depicted in tables 5 and 6.

To make a gel stick type formulation, Aluminium Chloride solution was poured into a beaker and calcium lactate was added. Stirring was adjusted to insure good mixing. Glyceryl stearate was added while mixing. Hydrogenated castor oil was added followed by the Bentone gel and the mixture was stirred until homogeneous. Amounts used are depicted in table 5.

TABLE 5: GEL-STICK TYPE ANTIPERSPIRANT COMPOSITION COMPRISING CALCIUM LACTATE (BENTONE GEL)

| Raw material | Weight (g) | RPM | Time (min) | %(w/w) |
|---|---|---|---|---|
| Aluminium Chloride solution | 600.00 |  |  | 60 |
| Ethanol denatured | 140.00 | 4000 | 2 | 14 |
| Calcium lactate | 100.00 | 4000 | 4 | 10 |
| Glyceryl stearate | 5.00 | 4000 | 3 | 0.5 |
| Hydrogenated Castor Oil | 5.00 | 4000 | 3 | 0.5 |
| Bentone gel IPM V | 150.00 | 4500 | 8 | 15 |
| sum | 1000.00 |  |  | 100 |
| RPM = rounds per minute. Time = the duration of stirring. | | | | |

In an alternative gel-stick formulation according to the invention, a 2 liter beaker was charged with ethanol and Hydroxypropylcellulose was sprinkled gently into the ethanol, while assuring moderate mixing. Calcium lactate was added and stirring was adjusted to insure good mixing. Glyceryl Stearate, Hydrogenated castor oil, and aluminium chloride solution were added in that order and the mixture was stirred until homogeneous. The silica was weighed in a separate beaker, and added slowly to the mixture, while stirring manually. When the silica had been wetted, the mixture was stirred, until homogeneous. Amounts used are depicted in table 6.

TABLE 6: GEL-STICK TYPE ANTIPERSPIRANT COMPOSITION COMPRISING CALCIUM LACTATE (SILICA)

| Raw material | Weight (g) | RPM | Time (min) | %(w/w) |
|---|---|---|---|---|
| Ethanol denatured | 239.25 |  |  | 23.925 |
| Hydroxypropylcellulose | 1.75 | 2500-4000 | 25 | 0.175 |
| Calcium lactate | 100.00 | 4000 | 4 | 10 |
| Glyceryl stearate | 4.50 | 4000 | 1 | 0.45 |
| Hydrogenated Castor Oil | 4.50 | 4000 | 3 | 0.45 |
| Aluminium Chloride solution | 600.00 | 4500 | 8 | 60 |

(continued)

| Raw material | Weight (g) | RPM | Time (min) | %(w/w) |
|---|---|---|---|---|
| Silica | 50.00 | 4500 | 3 | 5 |
| sum | 1000.00 | | | 100 |
| RPM = rounds per minute. Time = the duration of stirring. | | | | |

Example 3 - Efficacy studies

The purpose of this study was to assess the effectiveness of antiperspirant as defined in example 1 tables 1 and 2 in human subjects. A defined amount of the test material was applied to one axilla (right or left). The other axilla remained untreated. Each subject received five applications of the test material on the same axilla. Sweat secretion was determined gravimetrically before treatment (baseline) and after 48, 72, 96, and 120 hours after the last application. This investigation was carried out approximating the FDA-guidelines.

*Statistical data analysis*

The efficacy of the test product will be evaluated by calculating the pre-treatment and the post-treatment ratio for sweat secretion over two successive sweat collection periods of each subject. The post-treatment ratio of each subject is divided by the pre-treatment ratio to obtain an adjusted-treatment ratio (Z) for each subject:

$$Z = \frac{T * PC}{C * PT}$$

T (C) = post-treatment measure for the test (control) axilla,
PC (PT) = pre-treatment measure for the control (test) axilla,

The mean value of Z over all subjects is calculated. This value is used to evaluate the mean percentage sweat reduction.
Statistical analysis will be carried out using Wilcoxon signed-ranks test. Hypothesis testing is performed at the $\alpha$ = 0.05 level. Hypotheses tested to confirm standard antiperspirant efficacy (48, 72, 96 and 120 hours after last application) are:

$H_0$: Median Z $\geq$ 0.80, and
$H_A$: Median Z < 0.80

Rejection of the null hypothesis justifies the conclusion that at least 50 % of the target population will obtain a sweat reduction of at least 20 %, taking the FDA guideline as guide of reference.
Similar hypotheses are tested to confirm extra antiperspirant efficacy (48, 72, 96 and 120 hours after last application):

$H_0$: Median Z $\geq$ 0.70, and
$H_A$: Median Z < 0.70

Rejection of the null hypothesis justifies the conclusion that at least 50 % of the target population will obtain a sweat reduction of at least 30 %, taking the FDA guideline as guide of reference again.
The tolerability of the test product was assessed by objective and subjective dermatological evaluation. 55 subjects were screened for this study. 37 subjects were enrolled of which 36 subjects completed the study according to protocol or with minor deviations, i.e. Subject #34 withdrew his consent. Subject #14 and #27 did not get the 5th product application on Day 5. Measurements of Day 7, Day 8, and Day 9 correspond to 72 hours, 96 hours, and 120 hours after 4th application for these two subjects.

*Data treatment to demonstrate standard antiperspirant efficacy:*

The results of comparison to the benchmark 0.8 are presented in table 7.

TABLE 7: RESULTS OF ONE-SIDED WILCOXON SIGNED-RANKS TEST VERSUS 0.8 (CONTROL: B)

| Assessment | N | p-value of one-sided Wilcoxon Signed-Ranks Test $H_0$: Z $\geq$ 0.8 vs. $H_1$: Z < 0.8 |
|---|---|---|
| 48 h after 5th Appl. | 34 | <0.001 * |
| 72 h after 5th Appl. | 36 | <0.001 * |
| 96 h after 5th Appl. | 36 | <0.001 * |
| 120 h after 5th Appl. | 36 | <0.001 * |
| n.s.: not significant *: significant, two-sided p $\leq$ 0.025 | | |

Half of the tested panel had a decrease of sweat amount of at least 39 % for the test product A (according to example 1, tables 1 and 2) in comparison to untreated area (product B) 48, 72, 96, and 120 hours after the 5th application. The one-sided statistical comparison to benchmark 0.8 showed significant lower values for adjusted ratio Z regarding all assessments after the 5th application. The test demonstrates that, with high probability, at least 50 percent of the target population will obtain a sweat reduction of at least 20 percent.

As a result of the statistical analysis the test product (product A) can be designated as "effective" for 48, 72, 96, and 120 hours after the 5th application according to FDA guidelines (§ 350.60 (21 CFR 350.60) of the final monograph (final rule) for OTC antiperspirant drug products, published in the federal register on June 9, 2003).

*Data treatment to demonstrate extra-effective antiperspirant efficacy:*

Since the hypothesis $H_0$: Z $\geq$ 0.8 could be rejected second tests were performed with the hypothesis $H_0$: Z $\geq$ 0.7 which are presented in table 8.

TABLE 8: RESULTS OF ONE-SIDED WILCOXON SIGNED-RANKS TEST VERSUS 0.7 (CONTROL: B)

| Assessment | N | p-value of one-sided Wilcoxon Signed-Ranks Test $H_0$: Z $\geq$ 0.7 vs. $H_1$: Z < 0.7 |
|---|---|---|
| 48 h after 5th Appl. | 34 | <0.001 * |
| 72 h after 5th Appl. | 36 | 0.001 * |
| 96 h after 5th Appl. | 36 | <0.001 * |
| 120 h after 5th Appl. | 36 | 0.021 * |
| n.s.: not significant *: significant, two-sided p $\leq$ 0.025 | | |

The one-sided statistical comparison to benchmark 0.7 showed significant lower values for adjusted ratio Z regarding all assessments after the 5th application. The test demonstrates that, with high probability, at least 50 percent of the target population will obtain a sweat reduction of at least 30 percent.

As a result of the statistical analysis the test product (product A) can be designated as "extra effective" for 48, 72, 96, and 120 hours after the 5th application according to FDA guidelines.

In conclusion the composition of the present invention fulfilled the FDA "effectiveness" and "extra effectiveness" criteria at all measuring times, i.e. 48, 72, 96 and 120 h.

Example 4 - Comparative user evaluation studies on efficacy and skin feel

The composition according to the present invention (designated "new formulation" herein) has been subjected to comparative user evaluation studies, where it was compared to the prior art composition currently on the market as *Perspirex®* and disclosed in WO 2007/073740, example 1 (designated "existing type" herein).

Thus, the composition described in example 1, tables 1 and 2 (new formulation) was compared to a composition according to table 9. Thus, the major differences between the existing type and the new formulation, in terms of active product ingredients, are the replacement of aluminium lactate with calcium lactate in the new formulation, and the increase in aluminium chloride concentration in the new formulation.

TABLE 9: CURRENTLY MARKETED PRIOR ART COMPOSITION PERSPIREX® (EXISTING TYPE)

| Raw material | % (w/w) |
|---|---|
| Ethanol | 30-40 |
| Aluminium Chloride in ethanol 16,4% w/w | 40-50 |
| Aluminium Lactate | 8-20 |
| Cyclopentasiloxane | 5-10 |
| Hydrogenated microcrystalline wax | <1 |
| Cetyl Palmitate | <1 |
| Glyceryl Stearate | <1 |
| Hydrogenated Castor Oil | <1 |
| sum | 100 |

The studies were performed in Poland and Spain, where experienced users of the existing type antiperspirant composition were recruited (Spain N=19, Poland N=14). The results are depicted in tables 10-13 below.

TABLE 10: SCORING OF CHARACTERISTICS (SPAIN)

| To what degree do the listed sensory skin feeling characteristics occur during/after application  Score: 1: not at all, 2: a little, 3: moderately, 4: much, 5: very much (average is 3) | | |
|---|---|---|
| **Characteristic scored** | **Composition scored** | **Score** |
| Feels neutral/comfortable to the skin | Existing type | 3.3 |
| | new formulation | 3.4 |
| Feels greasy/sticky on the skin when applied | Existing type | 2.1 |
| | new formulation | 1.9 |
| Induces skin irritation (for example itching) | Existing type | 2.6 |
| | new formulation | 2.4 |

User preferences were also investigated according to table 11 below. The integers refers to number of users responding in the way given in the cell above, and the percentage is given as compared to total number of participants.

TABLE 11: COMPARATIVE USER EVALUATION (SPAIN)

| Sweat/Odour reducing effect How will you evaluate the new formulation compared to the existing type? | | |
|---|---|---|
| No difference | The new formulation was more effective | The new formulation was less effective |
| 6 (37.5%) | 6 (37.5%) | 4 (25%) |

(continued)

| Sensory skin feeling effect How will you evaluate the new formulation compared to the existing type? | | |
|---|---|---|
| No difference | The new formulation was improved/better | The new formulation was poorer |
| 6 (37.5%) | 8 (50%) | 2 (12.5 %) |
| Taking into consideration the sweat/odour reducing effect and the sensory skin feeling effect: do you prefer the new formulation or the existing type as your main antiperspirant in the future? | | |
| No difference | I prefer the existing type | I prefer the new formulation |
| 5 (31%) | 3 (19%) | 8 (50%) |

The analogous study in Poland yielded the results presented in tables 12-13:

TABLE 12: SCORING OF CHARACTERISTICS (POLAND)

| To what degree do the listed sensory skin feeling characteristics occur during/after application *Score: 1: not at all, 2: a little, 3: moderately, 4: much, 5: very much (average is 3)* | | |
|---|---|---|
| **Characteristic scored** | **Composition scored** | **Score** |
| Feels neutral/comfortable to the skin | Existing type | 2.3 |
| | new formulation | 2.2 |
| Feels greasy/sticky on the skin when applied | Existing type | 1.6 |
| | new formulation | 1.7 |
| Induces skin irritation (for example itching) | Existing type | 2.6 |
| | new formulation | 1.7 |

TABLE 13: COMPARATIVE USER EVALUATION (POLAND)

| Sweat/Odour reducing effect How will you evaluate the new formulation compared to the existing type? | | |
|---|---|---|
| No difference | The new formulation was more effective | The new formulation was less effective |
| 7 (58%) | 3 (25%) | 2 (17%) |
| Sensory skin feeling effect How will you evaluate the new formulation compared to the existing type? | | |
| No difference | The new formulation was improved/better | The new formulation was poorer |
| 2 (17%) | 9 (75%) | 1 (8.3%) |
| Taking into consideration the sweat/odour reducing effect and the sensory skin feeling effect: do you prefer the new formulation or the existing type as your main antiperspirant in the future? | | |
| No difference | I prefer the existing type | I prefer the new formulation |
| 1 (8.3%) | 1 (8.3%) | 10 (83.3%) |

The overall conclusion of the user evaluation study is that in terms of efficacy, i.e. sweat reduction and odour reducing effect, the majority of users considered the new formulation, i.e. the compositions of the present invention, either as good as, or better than the existing type.

With respect to a number of skin feel characteristics the new formulation has consistently scored better than the existing type (the two exceptions being the Spanish users considering the new formulation being slightly less neutral/comfortable to the skin and the Polish users considering the new formulation being slightly more greasy/sticky than the existing type). Particularly, with respect to the undesirable skin irritation symptoms (for example itching) new formulation performs surprisingly better than the existing type, particularly considering the elevated amount of aluminium chloride as compared to the existing type. Preliminary tests indicate that a new formulation comprising magnesium lactate also provides high efficacy in line with calcium lactate.

This demonstrates the further improved skin feel effects of calcium or magnesium salts of organic acids in this type of anhydrous composition, as compared to e.g. aluminium salts of organic acids.

Example 5 - Comparative efficacy tests

Comparative antiperspirant screening tests (36-ATS) were performed according to Good Clinical Practice (standard protocol-V03), wherein the existing type antiperspirant (product A) was compared to the new formulation as described in example 1, tables 1 and 2 (product B), the new formulation as described in example 2, table 5 (product E) and the new formulation comprising magnesium lactate as described in example 1, tables 3 and 4 (product H). The test was performed on 18-20 subjects, measuring the perspiration reducing efficacy on the back of the test subjects 72 h after application. The products were compared using a T-test and the results are given in table 14 below. Further, the new formulation (product B) was compared to an analogous composition comprising 2.5%(w/w) added water (product G) and product H with magnesium lactate. In product G the water simply replaces the same amount of ethanol.

TABLE 14: COMPARATIVE EFFICACY TESTS

| Product 1 | Product 2 | Difference Prd1-prd2 | Std. Error | t-value | DF | p-value | Result |
|---|---|---|---|---|---|---|---|
| A | B | 2.345 | 4.663 | 0.503 | 19 | 0.621 | n.s. |
| A | H | -2.099 | 4.426 | -0.474 | 18 | 0.641 | n.s. |
| A | E | -0.907 | 2.395 | -0.379 | 19 | 0.709 | n.s. |
| B | G | -3.228 | 3.889 | -0.830 | 20 | 0.416 | n.s. |
| B | H | -0.043 | 5.125 | -0.008 | 19 | 0.993 | n.s. |
| n.s.: not significant. "Prd" is an abbreviation for "Product". DF is an abbreviation for "Degrees of Freedom". | | | | | | | |

The comparative tests show that no significant reduction in sweat reducing efficacy can be detected when comparing the new formulations with the existing type formulation. Hence, the efficacy is maintained as compared to prior art compositions. It is further shown that the addition of 2.5%(w/w) water (Product G) does not influence efficacy significantly, indicating a tolerance for small amounts of water (<5%(w/w)) in the compositions. Also magnesium lactate shows the same efficacy as when using calcium lactate. It should be noted that the total amount of aluminium in the existing type product is about 11 % %(w/w) as a fair amount of aluminium is also derived from the aluminium lactate.

Example 6 - solution vs dispersion experiments

The inventors of the present invention made a series of compositions prepared according to the method disclosed in example 1 albeit without the addition of hydrogenated castor oil as castor oil does not dissolve in ethanol. It was hereby clearly demonstrated that the aluminium chloride and calcium lactate form a solution when prepared according to the invention and not a suspension. The compositions that were prepared according to the method of example 1 were documented by photos and are illustrated in figures 1-4.

Figure 1 shows an aluminum chloride solution in ethanol. The resulting composition is a clear liquid i.e. all aluminum chloride particles are solvated thereby forming a solution.

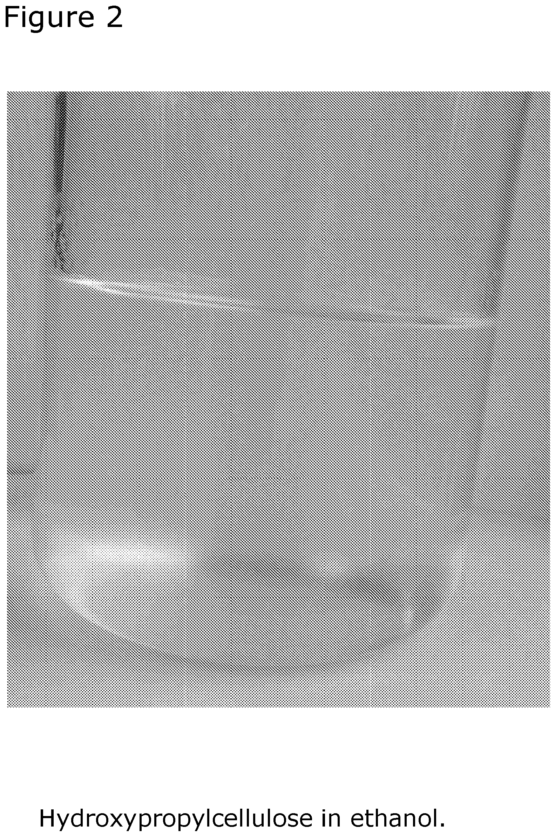

Figure 2 shows hydroxypropylcellulose in ethanol. The resulting composition was a slightly hazy liquid i.e. a mixture of hydroxypropylcellulose in ethanol alone does not provide a clear liquid albeit all hydroxypropylcellulose is solubilized.

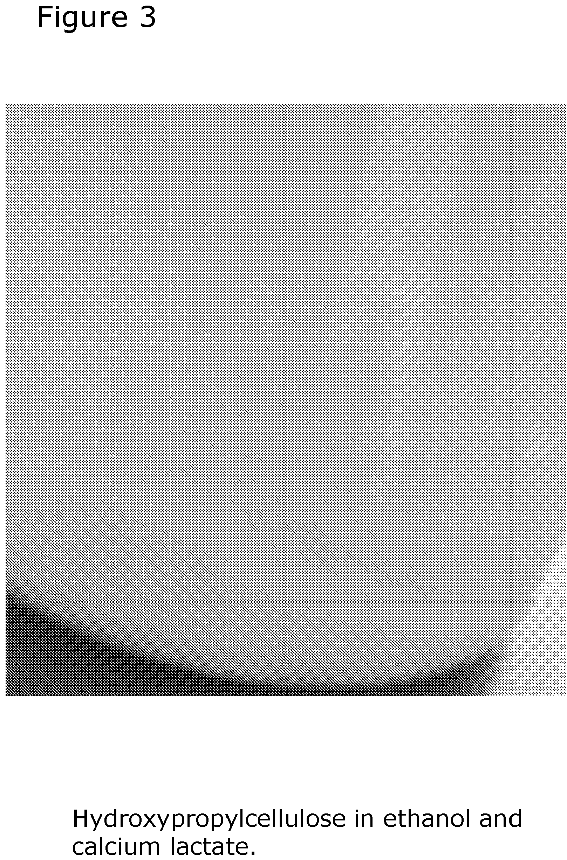

Figure 3 shows the composition depicted in figure 2 with the addition of calcium lactate. The compositions has been thoroughly mixed. The resulting composition is a hazy liquid i.e. a mixture comprising wetted but not solubilized calcium lactate.

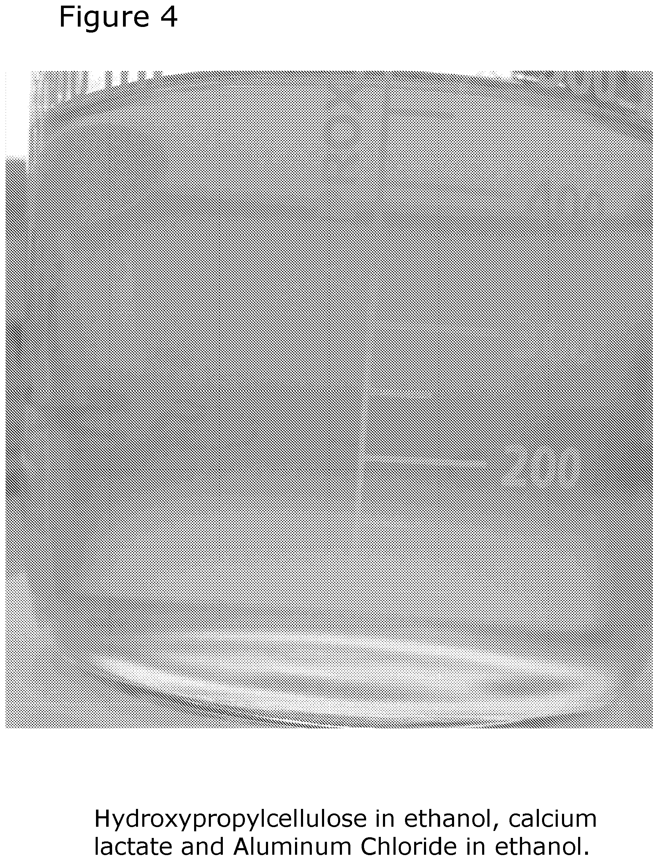

Figure 4 shows the composition depicted in figure 3 with the addition of the aluminum chloride solution in ethanol depicted in figure 1 i.e. the composition depicted in figure 4 consists of hydroxypropylcellulose in ethanol, calcium lactate and aluminium chloride in ethanol. It is clear that the addition of aluminium chloride in ethanol provides for the calcium lactate to dissolve entirely and that the slight haze in the finished product stems from the hydroxypropylcellulose (see figure 2).

Thus, overall the above figure demonstrate that a composition of the present invention comprising aluminum chloride and calcium lactate provides a solution/emulsion and not a suspension.

**References**

- WO 2007/073740
- EP 1105087
- US 3,998,788
- § 350.60 (21 CFR 350.60) of the final monograph (final rule) for OTC antiperspirant drug products, published in the federal register on June 9, 2003

**Claims**

1. An anhydrous antiperspirant composition comprising

    i) $AlCl_3$ or any hydrates thereof,
    ii) at least one calcium or magnesium salt of lactic acid, and
    iii) at least one water miscible solvent.

2. A composition according to claim 1, wherein said salt of a lactic acid is a calcium salt.

3. A composition according to any one of claims 1 or 2, wherein said salt of a lactic acid is calcium lactate.

4. A composition according to claim 1-3, wherein said composition comprises an oil phase.

5. A composition according to any one of claims 1-4, wherein said composition comprises a thickening agent.

6. A composition according to any one of claims 1-5, wherein said $AlCl_3$ or hydrate thereof is present in an amount in the range of 5-25%(w/w) as compared to total composition weight.

7. A composition according to any one of claims 1-6, wherein said calcium or magnesium salt of a lactic acid is present in an amount in the range of 1-25%(w/w) as compared to total composition weight.

8. A composition according to any one of claims 1-7, wherein said solvent is present in an amount in the range of 5-90% as compared to total composition weight.

9. A composition according to any one of claims 5-8, wherein said thickening agent is present in an amount in the range of 0.01-20% (w/w) as compared to total composition weight.

10. A composition according to any one of claims 4-9, wherein said oil phase is present in an amount in the range of 0.001-1.0% (w/w) as compared to total composition weight.

11. A composition according to any one of claims 1-10, wherein the $AlCl_3$ or any hydrates thereof and the calcium or magnesium salt of lactic acid are dissolved in the solvent.

12. A composition according to any one of claims 1-11, wherein said composition is a solution.

13. A composition according to any one of claims 1-12, wherein said composition has an efficacy which corresponds to an effectiveness where at least 50% of a group of efficacy test population experiences a more than 20% reduction in sweat production.

**14.** A method of preventing or reducing perspiration comprising applying to the skin an anhydrous antiperspirant composition comprising:

> i) AlCl$_3$ or any hydrates thereof,
> ii) at least one calcium or magnesium salt of lactic acid, and
> iii) at least one water miscible solvent.

**15.** A method of manufacturing an anhydrous antiperspirant composition comprising:

> I) providing at least one water miscible solvent
> II) optionally adding a thickening agent
> III) Adding at least one calcium or magnesium salt of lactic acid
> IV) optionally adding an oil phase
> V) Adding a solution of AlCl$_3$ or any hydrates thereof

to form said anhydrous antiperspirant composition.

**Patentansprüche**

**1.** Wasserfreie schweißhemmende Zusammensetzung, umfassend

> i) AlCl$_3$ oder ein beliebiges Hydrat davon,
> ii) zumindest ein Calcium- oder Magnesiumsalz von Milchsäure, und
> iii) zumindest ein wassermischbares Lösungsmittel.

**2.** Zusammensetzung nach Anspruch 1, wobei das Salz einer Milchsäure ein Calciumsalz ist.

**3.** Zusammensetzung nach einem der Ansprüche 1 oder 2, wobei das Salz einer Milchsäure Calciumlactat ist.

**4.** Zusammensetzung nach Anspruch 1-3, wobei die Zusammensetzung eine Ölphase umfasst.

**5.** Zusammensetzung nach einem der Ansprüche 1-4, wobei die Zusammensetzung ein Verdickungsmittel umfasst.

**6.** Zusammensetzung nach einem der Ansprüche 1-5, wobei das AlCl$_3$ oder das Hydrat davon in einer Menge in dem Bereich von 5-25 % (w/w) im Vergleich zum Gesamtgewicht der Zusammensetzung vorhanden ist.

**7.** Zusammensetzung nach einem der Ansprüche 1-6, wobei das Calcium- oder Magnesiumsalz einer Milchsäure in einer Menge in dem Bereich von 1-25 % (w/w) im Vergleich zum Gesamtgewicht der Zusammensetzung vorhanden ist.

**8.** Zusammensetzung nach einem der Ansprüche 1-7, wobei das Lösungsmittel in einer Menge in dem Bereich von 5-90 % im Vergleich zum Gesamtgewicht der Zusammensetzung vorhanden ist.

**9.** Zusammensetzung nach einem der Ansprüche 5-8, wobei das Verdickungsmittel in einer Menge in dem Bereich von 0,01-20 % (w/w) im Vergleich zum Gesamtgewicht der Zusammensetzung vorhanden ist.

**10.** Zusammensetzung nach einem der Ansprüche 4-9, wobei die Ölphase in einer Menge in dem Bereich von 0,001-1,0 % (w/w) im Vergleich zum Gesamtgewicht der Zusammensetzung vorhanden ist.

**11.** Zusammensetzung nach einem der Ansprüche 1-10, wobei das AlCl$_3$ oder ein beliebiges Hydrat davon und das Calcium- oder Magnesiumsalz von Milchsäure in dem Lösungsmittel gelöst sind.

**12.** Zusammensetzung nach einem der Ansprüche 1-11, wobei die Zusammensetzung eine Lösung ist.

**13.** Zusammensetzung nach einem der Ansprüche 1-12, wobei die Zusammensetzung eine Effizienz aufweist, die einer Wirksamkeit entspricht, bei der zumindest 50 % einer Gruppe von Effizienztestpopulation eine Reduzierung der Schweißproduktion um mehr als 20 % erfährt.

**14.** Verfahren zum Verhindern oder Reduzieren von Schweißbildung, umfassend ein Auftragen, auf die Haut, einer wasserfreien schweißhemmende Zusammensetzung, die Folgendes umfasst:

i) AlCl$_3$ oder ein beliebiges Hydrat davon,
ii) zumindest ein Calcium- oder Magnesiumsalz von Milchsäure, und
iii) zumindest ein wassermischbares Lösungsmittel.

**15.** Verfahren zum Herstellen einer wasserfreien schweißhemmende Zusammensetzung, umfassend:

I) Bereitstellen von zumindest einem wassermischbaren Lösungsmittel
II) optionales Hinzufügen eines Verdickungsmittels
III) Hinzufügen von zumindest einem Calcium- oder Magnesiumsalz von Milchsäure
IV) optionales Hinzufügen einer Ölphase
V) Hinzufügen einer Lösung von AlCl$_3$ oder einem beliebigen Hydrat davon, um die wasserfreie schweißhemmende Zusammensetzung zu bilden.

**Revendications**

**1.** Composition antiperspirante anhydre comprenant

i) de l'AlCl$_3$ ou tout hydrate de celui-ci,
ii) au moins un sel de calcium ou de magnésium d'acide lactique, et
iii) au moins un solvant miscible à l'eau.

**2.** Composition selon la revendication 1, ledit sel d'acide lactique étant un sel de calcium.

**3.** Composition selon l'une quelconque des revendications 1 ou 2, ledit sel d'acide lactique étant le lactate de calcium.

**4.** Composition selon les revendications 1 à 3, ladite composition comprenant une phase huileuse.

**5.** Composition selon l'une quelconque des revendications 1 à 4, ladite composition comprenant un agent épaississant.

**6.** Composition selon l'une quelconque des revendications 1 à 5, ledit AlCl$_3$ ou hydrate de celui-ci étant présent en une quantité se trouvant dans la plage de 5 à 25 % (p/p) par rapport au poids total de la composition.

**7.** Composition selon l'une quelconque des revendications 1 à 6, ledit sel de calcium ou de magnésium d'acide lactique étant présent en une quantité se trouvant dans la plage de 1 à 25 % (p/p) par rapport au poids total de la composition.

**8.** Composition selon l'une quelconque des revendications 1 à 7, ledit solvant étant présent en une quantité se trouvant dans la plage de 5 à 90 % par rapport au poids total de la composition.

**9.** Composition selon l'une quelconque des revendications 5 à 8, ledit agent épaississant étant présent en une quantité se trouvant dans la plage de 0,01 à 20 % (p/p) par rapport au poids total de la composition.

**10.** Composition selon l'une quelconque des revendications 4 à 9, ladite phase huileuse étant présente en une quantité se trouvant dans la plage de 0,001 à 1,0 % (p/p) par rapport au poids total de la composition.

**11.** Composition selon l'une quelconque des revendications 1 à 10, ledit AlCl$_3$ ou tout hydrate de celui-ci et le sel de calcium ou de magnésium d'acide lactique étant dissous dans le solvant.

**12.** Composition selon l'une quelconque des revendications 1 à 11, ladite composition étant une solution.

**13.** Composition selon l'une quelconque des revendications 1 à 12, ladite composition présentant une efficacité qui correspond à une efficacité dans laquelle au moins 50 % d'un groupe d'une population soumise à un test d'efficacité connaît une réduction de plus de 20 % dans la production de sueur.

**14.** Procédé permettant d'empêcher ou de réduire la transpiration comprenant l'application d'une composition antiperspirante anhydre comprenant :

i) de l'AlCl$_3$ ou tout hydrate de celui-ci,
ii) au moins un sel de calcium ou de magnésium d'acide lactique, et
iii) au moins un solvant miscible à l'eau.

15. Procédé de fabrication d'une composition antiperspirante anhydre comprenant :

I) la fourniture d'au moins un solvant miscible à l'eau
II) l'ajout éventuel d'un agent épaississant
III) l'ajout d'au moins un sel de calcium ou de magnésium d'acide lactique
IV) l'ajout éventuel d'une phase huileuse
V) l'ajout d'une solution d'AlCl$_3$ ou de tout hydrate de celui-ci

pour former ladite composition antiperspirante anhydre.

# Figure 1

Aluminum Chloride in ethanol.

# Figure 2

Hydroxypropylcellulose in ethanol.

# Figure 3

Hydroxypropylcellulose in ethanol and calcium lactate.

# Figure 4

Hydroxypropylcellulose in ethanol, calcium lactate and Aluminum Chloride in ethanol.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007073740 A
- EP 1105087 A
- US 3998788 A